(19) Europäisches Patentamt
European Patent Office
Office européen des brevets

(11) Veröffentlichungsnummer: **0 033 120**
**B1**

(12) # EUROPÄISCHE PATENTSCHRIFT

(45) Veröffentlichungstag der Patentschrift: **29.06.83**

(51) Int. Cl.³: **C 07 J 1/00**, C 12 P 33/06, A 61 K 31/565

(21) Anmeldenummer: **81100381.3**

(22) Anmeldetag: **20.01.81**

(54) **1-Hydroxysteroide, Verfahren zu ihrer Herstellung und pharmazeutische Präparate, die diese Verbindungen enthalten.**

(30) Priorität: **23.01.80 DE 3002746**

(43) Veröffentlichungstag der Anmeldung: **05.08.81 Patentblatt 81/31**

(45) Bekanntmachung des Hinweises auf die Patenterteilung: **29.06.83 Patentblatt 83/26**

(84) Benannte Vertragsstaaten: **AT BE CH DE FR GB IT LI LU NL SE**

(56) Entgegenhaltungen:
**DE-A-2 614 340**
**FR-A-2 287 909**
**FR-A-2 326 927**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Petzoldt, Karl, Dr., Flachsweg 10, D-1000 Berlin 38 (DE)**
Erfinder: **Wiechert, Rudolf, Prof. Dr., Petzower Strasse 8 a, D-1000 Berlin 39 (DE)**
Erfinder: **Steinbeck, Hermann, Dr., Hammerstrasse 46, D-1000 Berlin 37 (DE)**
Erfinder: **Elger, Walter, Dr., Schorlemer, Allee 12 B, D-1000 Berlin 33 (DE)**

**EP 0 033 120 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 1-Hydroxysteroide, Verfahren zu ihrer Herstellung und pharmazeutische Präparate, die diese Verbindungen enthalten

Die Erfindung betrifft den in den Patentansprüchen gekennzeichneten Gegenstand.

Aus der FR-A Nr. 2287909 sind die entsprechenden in 15,16-Stellung gesättigten Steroide und aus der FR-A Nr. 2326927 die entsprechenden in 1-Stellung nicht hydroxylierten Steroide bekannt. Diese bekannten Steroide zeigen bereits eine starke gestagene Wirkung. Es wurde nun gefunden, dass die erfindungsgemässen 1-Hydroxysteroide der allgemeinen Formel I des Anspruchs 1 sowohl gegenüber den in 15,16-Stellung gesättigten als auch gegenüber den in 1-Stellung nicht hydroxylierten Verbindungen eine stärkere gestagene Hauptwirkung und eine geringere androgenen Nebenwirkung aufweisen.

In Formel I können die Gruppen $R_1$ und $R_2$ gleich oder verschieden sein und Wasserstoff oder Alkanoylgruppen mit 1 bis 8 Kohlenstoffatomen bedeuten. Namentlich genannt seien die Formyl-, Acetyl, Propionyl-, Butyryl-, Pentanoyl-, Hexanoyl-, Heptanoyl- und Octanoylgruppe.

Die neuen 1-Hydroxysteroide werden nach dem Verfahren gemäss Anspruch 8 hergestellt. Das Verfahren lässt sich beispielsweise unter Verwendung folgender Pilzstämme durchführen:

*Aspergillus clavatus* (ATCC 9598)
*Calonectria decora* (ATCC 14767)
*Mucor griseocyanus* (ATCC 1207 b)
*Glomerella glycines* (ATCC 11871)
*Septomyxa affinis* (ATCC 6737)

Bevorzugt geeignet ist der Stamm *Aspergillus clavatus*.

Die Durchführung der Hydroxylierung erfolgt nach Methoden, welche man üblicherweise zur mikrobiologischen Hydroxylierung von Steroiden mit Pilzkulturen anwendet.

So werden zunächst in allgemein üblichen Vorversuchen die günstigsten Fermentationsbedingungen, wie zum Beispiel Auswahl des günstigsten Nährmediums, des geeigneten Substratlösungsmittels, der Substratskonzentration, der technischen Bedingungen — wie Temperatur, Belüftung, pH — und der optimalen Zeiten für Germination, Substratzugabe und Substratkontakt am Enzym des Mikroorganismus analytisch, insbesondere dünnschichtchromatographisch, ermittelt.

Dabei hat sich gezeigt, dass es zweckmässig ist, Konzentrationen von etwa 50-1000 mg Substrat pro l Nährmedium einzusetzen. Der pH-Wert wird vorzugsweise auf einen Wert im Bereich von 5 bis 7 eingestellt. Die Züchtungstemperatur liegt im Bereich von 20 bis 40° C, vorzugsweise von 25 bis 35° C. Zur Belüftung werden etwa 1 l Luft/min/l Kulturbrühe zugeführt. Die Umwandlung des Substrats wird zweckmässigerweise durch dünnschichtchromatographische Analyse von Probeextrakten verfolgt. Im allgemeinen haben sich nach 20 bis 120 h ausreichende Mengen an hydroxyliertem Steroid gebildet.

Nach erfolgter Fermentation werden die Fermentationsprodukte in an sich bekannter Weise isoliert. Die Isolierung kann zum Beispiel in der Weise erfolgen, dass man die Fermentationsansätze mit einem polaren, nicht wasserlöslichen Lösungsmittel, wie Äthylacetat, Butylacetat oder Methylisobutylketon, extrahiert, die Extrakte eindampft und die so erhalten Rohprodukte gegebenenfalls durch Chromatographie und/oder Kristallisation reinigt.

Die sich gegebenenfalls anschliessende Veresterung der freien Hydroxygruppen erfolgt nach den Methoden, die man üblicherweise in der Steroidchemie zur Veresterung sekundärer und tertiärer Hydroxygruppen verwendet. Als geeignete Veresterungsmethode sei beispielsweise die Umsetzung der Steroide mit Säureanhydriden oder Säurechloriden in Gegenwart basischer Katalysatoren, wie Natriumhydrogenkarbonat, Kaliumhydrogenkarbonat, Kaliumkarbonat, Natriumhydroxid, Kaliumhydroxid, Pyridin, Lutidin, Collidin oder 4-Dimethylaminopyridin, genannt. Je nach Wahl der Reaktionsbedingungen und Reaktionszeit gelangt man zu den 1-Mono- oder 1,17-Diacylsteroiden. So werden bei Reaktionstemperaturen von 0-30° C innerhalb von 10 bis 20 h vorwiegend die Monoacyl- und bei Reaktionstemperaturen von 10-50° C innerhalb von 20 bis 100 h die Diacylsteroide erhalten.

Die neuen 1-Hydroxysteroide der allgemeinen Formel I sind pharmakologisch wirksame Substanzen. Sie zeigen ein ähnliches Wirkungsspektrum wie die in 1-Stellung nicht hydroxylierten Steroide. So sind die Verbindungen der allgemeinen Formel I durch eine starke gestagene Wirkung ausgezeichnet. Beispielsweise erweist sich 17α-äthinyl-1α,17-dihydroxy-18-methyl-4,15-östradien-3-on (A) im üblichen Clauberg-Test dem nicht hydroxylierten 17α-Äthinyl-17-hydroxy-18-methyl-4,15-östradien-3-on (B) überlegen.

In der folgenden Tabelle werden die McPhail-Werte nach oraler Applikation an infantilen weiblichen Kaninchen angegeben.

*Clauberg-Test p.o.*

| Verbindung | Dosis (mg) | McPhail |
|---|---|---|
| A | 0,03 | 3,1 |
| | 0,01 | 1,9 |
| B | 0,03 | 2,9 |
| | 0,01 | 1,6 |

Die Bestimmung erfolgte nach der McPhail-Skala. (Beurteilungsgrade 1-4; 1 = keine Umwandlung des Endometriums, 4 = vollständige Umwandlung des Endometriums.)

Darüber hinaus haben die neuen 1-Hydroxysteroide nur eine sehr geringe androgene Nebenwirkung.

Die höheren Ester der erfindungsgemässen Verbindungen zeichnen sich durch protrahierte Wirksamkeit aus.

Die Verbindungen können zum Beispiel in Antikonzeptionspräparaten Verwendung finden, wobei sie als Gestagenkomponente in Kombination mit einer östrogenwirksamen Hormonkomponente, wie zum Beispiel Äthinylöstradiol, oder als alleinige Wirkkomponente eingesetzt werden. Die Verbindungen können aber auch in Präparaten zur Behandlung gynäkologischer Störungen eingesetzt werden.

Zum Gebrauch werden die neuen Verbindungen mit den in der galenischen Pharmazie üblichen Zusätzen, Trägersubstanzen und Geschmackskorrigentien nach an sich bekannten Methoden zu den üblichen Arzneimittelformen verarbeitet. Für die orale Applikation kommen insbesondere Tabletten, Dragées, Kapseln, Pillen, Suspensionen oder Lösungen in Frage. Für die parenterale Applikation kommen insbesondere ölige Lösungen, wie zum Beispiel Sesamöl- oder Rizinusöllösungen in Frage, die gegebenenfalls zusätzlich noch ein Verdünnungsmittel, wie zum Beispiel Benzylbenzoat oder Benzylalkohol, enthalten können. Die Konzentration des Wirkstoffes ist abhängig von der Applikationsform. So enthalten beispielsweise Tabletten zur oralen Applikation vorzugsweise 0,01-0,5 mg Wirkstoff und Lösungen zur parenteralen Applikation vorzugsweise 1-100 mg Wirkstoff pro 1 ml Lösung.

Die Dosierung der erfindungsgemässen Arzneimittel kann sich mit der Form und dem Zweck der Verabfolgung ändern. Beispielsweise liegt die tägliche kontrazeptive Dosis bei oraler Applikation bei 0,01-0,5 mg.

*Beispiel 1:*

Ein 2-l-Erlenmeyerkolben, der 500 ml einer 30 min bei 120° C im Autoklaven sterilisierten Nährlösung aus 3,0% Glucose, 1,0% Cornsteep liquor, 0,2% $NaNO_3$, 0,1% $KH_2PO_4$, 0,2% $K_2HPO_4$, 0,05% $MgSO_4 \cdot 7\,H_2O$, 0,002% $FeSO_4 \cdot 7\,H_2O$ und 0,05% KCl enthält, wird mit einer Schrägröhrchenkultur des Stammes *Aspergillus clavatus* (ATCC 9598) beimpft und 2½ d auf einem Rotationsschüttler geschüttelt. Mit 250 ml dieser Anzuchtkultur wird dann ein 20-l-Vorfermenter beimpft, der mit 15 l eines 60 min bei 121° C und 1,1 atü sterilisierten Mediums der gleichen Zusammensetzung wie die Anzuchtkultur gefüllt ist. Unter Zugabe von Silicon SH als Antischaummittel wird bei 29° C und 0,7 atü Druck unter Belüftung (10 l/min) und Rühren (220 tr/min) 24 h germiniert. Danach werden 900 ml dieser Kultur unter sterilen Bedingungen entnommen und damit ein 20-l-Hauptfermenter beimpft, der mit 14 l eines wie oben sterilisierten Nährmediums der gleichen Zusammensetzung wie die Vorfermenterkultur beschickt ist. Nach einer Anwachsphase von 12 h unter Vorfermenterbedingungen werden 3 g 17α-Äthinyl-17-hydroxy-18-methyl-4,15-östradien-3-on, gelöst in 150 ml Dimethylformamid, unter sterilen Bedingungen hinzugegeben und weiter gerührt und belüftet. Der Verlauf der Fermentation wird durch Entnahme von Proben kontrolliert, die mittels Methylisobutylketon extrahiert und dünnschichtchromatographisch analysiert werden. Nach 92 h Kontaktzeit ist die Umsetzung des Substrates beendet. Der Fermenterinhalt wird zweimal mit je 10 l Methylisobutylketon extrahiert, die Extrakte vereinigt und zunächst im Umlaufverdampfer konzentriert, anschliessend bei 50° C Badtemperatur im Vakuum im Rotationsverdampfer zur Trockne eingeengt. Den Rückstand löst man in warmen Methanol, filtriert vom ungelöst gebliebenen Siliconöl ab, dampft wieder zur Trockne ein und chromatographiert über eine Kieselgelsäule (Gradientelution: Hexan-Hexan/Essigester 1:1). Danach löst man das Produkt in wenig Benzol in der Siedehitze auf und lässt langsam abkühlen, wobei die 1α-Hydroxy-Verbindungen als reines kristallines Solvat (1,56 g) vom Schmelzpunkt 129-130° C mit 0,5 mol Benzol auskristallisiert. Zur Spaltung des Benzolsolvats wird die Substanz in wenig Äthanol gelöst, bis zur ersten Trübung mit destilliertem Wasser versetzt und das Benzol im Vakuum azeotrop abdestilliert. Anschliessend wird die klare Lösung gefriergetrocknet, wobei das 17α-Äthinyl-1α, 17-dihydroxy-18-methyl-4,15-östradien-3-on in Form eines mehlig-amorphen, sehr gut applizierbaren Produkts anfällt.

*Beispiel 2:*

200 mg 17α-Äthinyl-1α,17-dihydroxy-18-methyl-4,15-östradien-3-on werden in 10 ml Pyridin gelöst und unter Eiskühlung tropfenweise mit 3 ml Acetanhydrid versetzt. Dann lässt man die Lösung erwärmen und rührt bei Raumtemperatur über Nacht. Danach wird das Reaktionsgemisch in Eiswasser eingerührt, der ausgefällte ölig-kristalline Niederschlag abgesaugt, in Essigester aufgenommen, neutralgewaschen, über $Na_2SO_4$ getrocknet und in Vakuum zur Trockne eingeengt. Der verbliebene Rückstand wird aus Äther/Hexan kristallisiert. Man erhält 165 mg 1α-Acetoxy-17α-äthinyl-17-hydroxy-18-methyl-4,15-östradien-3-on vom Schmelzpunkt 123-126° C.

*Beispiel 3:*

Unter den Bedingungen des Beispiels 2 werden 200 mg 17α-Äthinyl-1α,17-dihydroxy-18-methyl-4,15-östradien-3-on mit Buttersäureanhydrid umgesetzt, wobei 140 mg 17α-Äthinyl-1α-butyryloxy-17-hydroxy-18-methyl-4,15-östradien-3-on von öliger Konsistenz erhalten werden.

*Beispiel 4:*

Unter den Bedingungen des Beispiels 2 werden 200 mg 17α-Äthinyl-1α,17-dihydroxy-18-methyl-4,15-östradien-3-on mit Caprylsäureanhydrid umgesetzt, wobei 130 mg 17α-Äthinyl-17-hydroxy-18-methyl-1α-octanoyloxy-4,15-östradien-3-on von öliger Konsistenz erhalten werden.

*Beispiel 5:*

200 mg 17α-Äthinyl-1α,17-dihydroxy-18-methyl-4,15-östradien-3-on werden mit 10 ml Pyridin und 3 ml Acetanhydrid versetzt und 4 d bei Raumtemperatur unter Stickstoff gerührt. Danach wird die Reaktionsmischung im Hochvakuum eingedampft, der Rückstand in Essigsäureäthylester aufgenommen und mit destilliertem Wasser neutralgewaschen. Nach dem Trocknen über Natriumsulfat und Einengen der Essigesterlösung im Vakuum erhält man 17α-Äthinyl-1α,17-diacetoxy-18-methyl-4,15-östradien-3-on als Öl.

*Beispiel 6* (Zusammensetzung einer Tablette)

| | |
|---|---|
| 0,075 mg | 17α-Äthinyl-1α, 17-dihydroxy-18-methyl-4,15-östradien-3-on |
| 0,030 mg | 17α-Äthinylöstradiol |
| 109,895 mg | Milchzucker (DAB 6) |
| 8,000 mg | Maisstärke (USP XVI) |
| 1,000 mg | Magnesiumstearat (USP XVI) |
| 1,000 mg | Talkum |
| 120,000 mg | Gesamtgewicht der Tablette |

*Beispiel 7* (Zusammensetzung eines Dragées)

| | |
|---|---|
| 0,100 mg | 17α-Äthinyl-1α, 17-dihydroxy-18-methyl-4,15-östradien-3-on |
| 0,020 mg | 17α-Äthinylöstradiol |
| 31,880 mg | Milchzucker |
| 18,425 mg | Maisstärke |
| 2,060 mg | Polyvinylpyrrolidon 25 |
| 0,010 mg | p-Oxybenzoesäuremethylester |
| 0,005 mg | p-Oxybenzoesäurepropylester |
| 2,500 mg | Talkum |
| 55,000 mg | Gesamtgewicht der Tablette, die mit üblicher Zuckermischung auf etwa 90 mg dragiert wird. |

*Beispiel 8* (Zusammensetzung einer Tablette)

| | |
|---|---|
| 0,100 mg | 17α-Äthinyl-1α, 17-dihydroxy-18-methyl-4,15-östradien-3-on |
| 63,600 mg | Milchzucker |
| 15,000 mg | mikrokristalline Cellulose |
| 1,000 mg | Talkum |
| 0,300 mg | Magnesiumstearat |
| 80,000 mg | Gesamtgewicht der Tablette |

**Patentansprüche für die Vertragsstaaten: BE, CH, DE, FR, GB, IT, LI, LU, NL, SE**

1. 1-Hydroxysteroide der allgemeinen Formel (I)

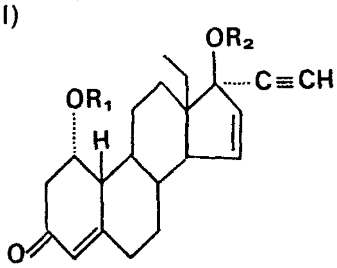

(I)

worin

$R_1$ und $R_2$ Wasserstoffatome oder Alkanoylgruppen mit 1 bis 8 Kohlenstoffatomen bedeuten.

2. 17α-Äthinyl-1α,17-dihydroxy-18-methyl-4,15-östradien-3-on.

3. 1α-Acetoxy-17α-äthinyl-17-hydroxy-18-methyl-4,15-östradien-3-on.

4. 17α-Äthinyl-1α-butyryloxy-17-hydroxy-18-methyl-4,15-östradien-3-on.

5. 17α-Äthinyl-17-hydroxy-18-methyl-1α-octanoyloxy-4,15-östradien-3-on.

6. 17α-Äthinyl-1α,17-diacetoxy-18-methyl-4,15-östradien-3-on.

7. Pharmazeutische Präparate, gekennzeichnet durch einen Gehalt an Verbindungen gemäss den Ansprüchen 1 bis 6.

8. Verfahren zur Herstellung von 1-Hydroxysteroiden der allgemeinen Formel (I)

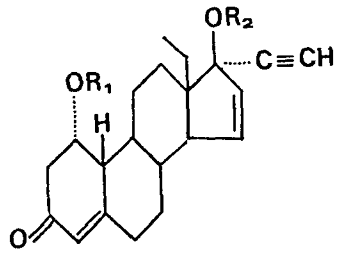

(I)

worin

$R_1$ und $R_2$ Wasserstoffatome oder Alkanoylgruppen mit 1 bis 8 Kohlenstoffatomen bedeuten, dadurch gekennzeichnet, dass man eine Verbindung der allgemeinen Formel (II)

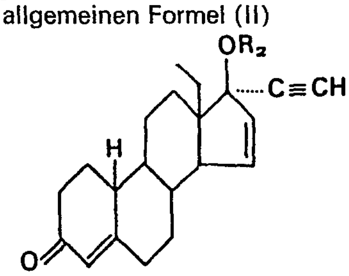

(II)

worin

$R_2$ die oben angegebene Bedeutung hat, mit einer Pilzkultur der Gattungen *Aspergillus, Calonectria, Mucor, Glomerella* oder *Septomyxa* fermentiert und gewünschtenfalls freie Hydroxygruppen verestert.

**Patentanspruch für den Vertragsstaat AT**

Verfahren zur Herstellung von 1-Hydroxysteroiden der allgemeinen Formel (I)

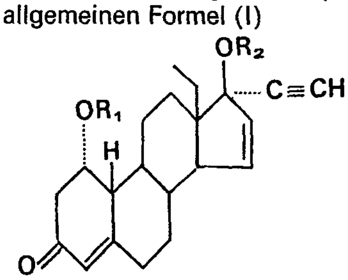

(I)

worin

$R_1$ und $R_2$ Wasserstoffatome oder Alkanoylgruppen mit 1 bis 8 Kohlenstoffatomen bedeuten, dadurch gekennzeichnet, dass man eine Verbindung der allgemeinen Formel (II)

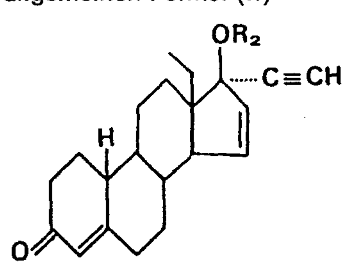

(II)

worin

$R_2$ die oben angegebene Bedeutung hat, mit einer Pilzkultur der Gattungen *Aspergillus, Calonectria, Mucor, Glomerella* oder *Septomyxa* fermentiert und gewünschtenfalls freie Hydroxygruppen verestert.

**Claim for the contracting State AT**

Process for the manufacture of 1-hydroxysteroids of the general formula (I)

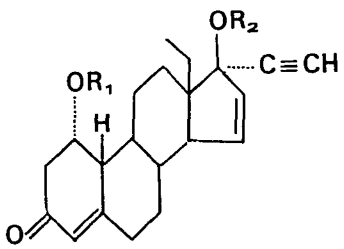

(I)

in which $R_1$ and $R_2$ represent hydrogen atoms or alkanoyl groups characterised in that a compound of the general formula (II)

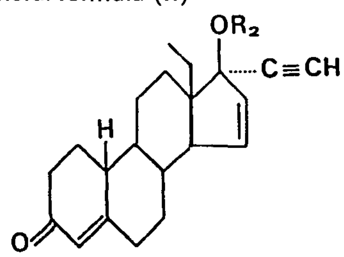

(II)

in which $R_2$ has the meaning given above, is fermented with a fungal culture of the genera *Aspergillus, Calonectria, Mucor, Glomerella* or *Septomyxa* and, if desired, free hydroxy groups are esterified.

**Claims for the contracting States BE, CH, DE, FR, GB, IT, LI, LU, NL, SE**

1. 1-Hydroxysteroids of the general formula (I)

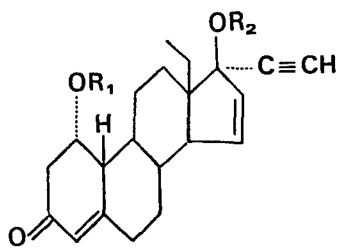

(I)

in which $R_1$ and $R_2$ represent hydrogen atoms or alkanoyl groups having from 1 to 8 carbon atoms.

2. 17α-Ethynyl-1α,17-dihydroxy-18-methyl-4,15-estradien-3-one.

3. 1α-Acetoxy-17α-ethynyl-17-hydroxy-18-methyl-4,15-estradien-3-one.

4. 17α-Ethynyl-1α-butyryloxy-17-hydroxy-18-methyl-4,15-estradien-3-one.

5. 17α-Ethynyl-17-hydroxy-18-methyl-1α-octanoyloxy-4,15-estradien-3-one.

6. 17α-Ethynyl-1α,17-diacetoxy-18-methyl-4,15-estradien-3-one.

7. Pharmaceutical preparations, characterised by a content of compounds according to claims 1 to 6.

8. Process for the manufacture of 1-hydroxysteroids of the general formula (I)

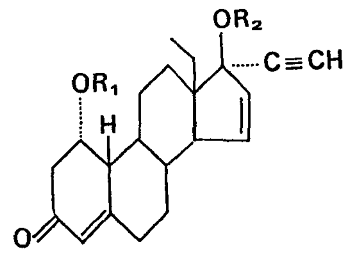

(I)

in which $R_1$ and $R_2$ represent hydrogen atoms or alkanoyl groups having from 1 to 8 carbon atoms, characterised in that a compound of the general formula (II)

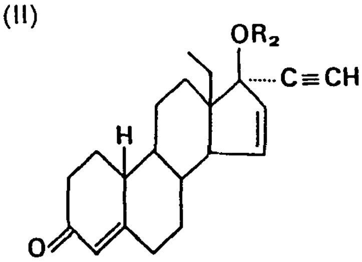

(II)

in which $R_2$ has the meaning given above, is fermented with a fungal culture of the genera *Aspergillus, Calonectria, Mucor, Glomerella* or *Septomyxa* and, if desired, free hydroxy groups are esterified.

**Revendication pour l'Etat contractant: AT**

Procédé de préparation de 1-hydroxystéroïdes répondant à la formule générale (I)

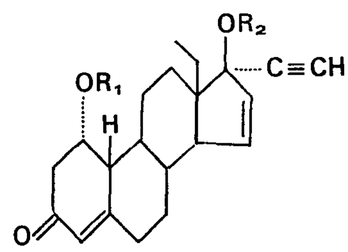

(I)

dans laquelle $R_1$ et $R_2$ représentent des atomes d'hydrogène ou des groupes alcanoyle comprenant 1 à 8 atomes de carbone, caractérisé en ce qu'on fait fermenter un composé répondant à la formule générale (II)

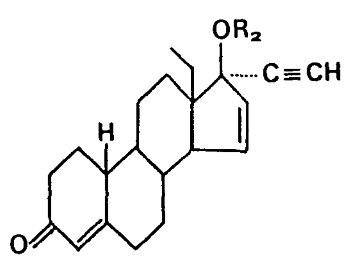

(II)

dans laquelle $R_2$ a la même signification que celle donnée ci-dessus, par une culture de champignon des genres *Aspergillus, Calonectria, Mucor, Glomerella* ou *Septomyxa*, et en ce que, éventuellement, on estérifie des groupes hydroxy libres.

**Revendications pour les Etats contractants BE, CH, DE, FR, GB, IT, LI, LU, NL, SE**

1. 1-Hydroxystéroïdes répondant à la formule générale (I)

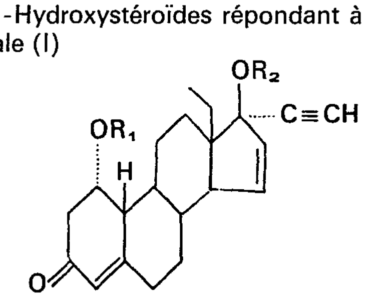

(I)

dans laquelle $R_1$ et $R_2$ représentent des atomes d'hydrogène ou des groupes alcanoyle comprenant de 1 à 8 atomes de carbone.

2. La 17α-éthynyl-1α,17-dihydroxy-18-méthyl-4,15-œstradién-3-one.

3. La 1α-acétoxy-17α-éthynyl-17-hydroxy-18-méthyl-4,15-œstradién-3-one.

4. La 17α-éthynyl-1α-butyryloxy-17-hydroxy-18-méthyl-4,15-œstradién-3-one.

5. La 17α-éthynyl-17-hydroxy-18-méthyl-1α-octanoyloxy-4,15-œstradién-3-one.

6. La 17α-éthynyl-1α,17-diacétoxy-18-méthyl-4,15-œstradién-3-one.

7. Compositions pharmaceutiques, caractérisées par une teneur en composés suivant l'une des revendications 1 à 6.

8. Procédé de préparation de 1-hydroxystéroïdes répondant à la formule générale (I)

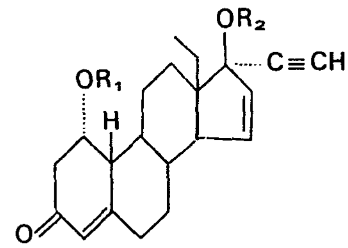

(I)

dans laquelle $R_1$ et $R_2$ représentent des atomes d'hydrogène ou des groupes alcanoyle comprenant 1 à 8 atomes de carbone, caractérisé en ce qu'on fait fermenter un composé répondant à la formule générale (II)

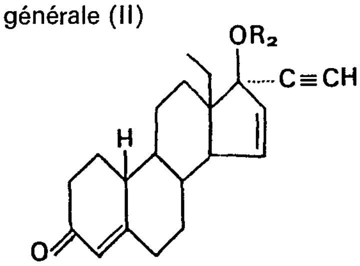

(II)

dans laquelle $R_2$ a la même signification que celle donnée ci-dessus, par une culture de champignon des genres *Aspergillus, Calonectria, Mucor, Glomerella* ou *Septomyxa*, et en ce que, éventuellement, on estérifie des groupes hydroxy libres.